# EUROPÄISCHE PATENTSCHRIFT

(11) **EP 0 614 881 B1**
(45) Veröffentlichungstag und Bekanntmachung des Hinweises auf die Patenterteilung: **05.11.1997**
(21) Anmeldenummer: 94103024.9
(22) Anmeldetag: 01.03.1994
(51) Int. Cl.: C07C 213/08, C07H 15/12

(54) **Verfahren zur Herstellung von tertiären Dialkylpolyhydroxyaminen**
Method for the production of tertiary dialkylpolyhydroxyamines
Procédé pour la préparation de dialkylpolyhydroxyamines tertiaires

(30) Priorität: 06.03.1993 DE 4307163
(43) Veröffentlichungstag der Anmeldung: 14.09.1994
(73) Patentinhaber: Clariant GmbH, 65929 Frankfurt am Main (DE)
(72) Erfinder: Weinelt, Frank, Dr., D-84508 Burgkirchen (DE)

(56) Entgegenhaltungen:
- EP-A- 0 142 868
- FR-A- 2 203 382
- GB-A- 908 203
- US-A- 4 190 601

## Beschreibung

Tertiäre Dialkylpolyhydroxyamine, insbesondere in Form von Dialkylglycaminen wie Dimethylglucamin, sind wertvolle Verbindungen, zum Beispiel als Zusatz zu Benetzungsmitteln und Reinigungsmitteln, zu Weichmachern, zu Gleit- und Schmiermitteln und dergleichen. Gemäß US-Patentschrift 2,016,962 erfolgt ihre Herstellung durch Umsetzung von einem reduzierenden Zucker wie Glucose, Fructose, Lactose oder Xylose mit einem Dialkylamin unter hydrierenden Bedingungen. Im einzelnen wird so vorgegangen, daß eine Zuckerverbindung mit einem Dialkylamin in Wasser als Lösungsmittel und in Gegenwart von einem Nickel-Trägerkatalysator bei einem Wasserstoffdruck von mindestens 15 bar und einer Temperatur von 80 bis 125 °C zu Dialkylpolyhydroxyamin umgesetzt wird. Dieses schon seit langem bekannte Verfahren (das ist die reduzierende Alkylierung von Zuckerverbindungen mit einem Dialkylamin) hat den Nachteil, daß das tertiäre Zuckeramin im allgemeinen in geringer Ausbeute erhalten wird.

In EP-A-0 142 868 wird ein Verfahren zur Herstellung von tertiären Alkylaminen beschrieben durch hydrierende Umsetzung von einem sekundären Alkylamin und einer Aldehydverbindung. Die Umsetzung wird - ganz im Gegensatz zum beanspruchten Verfahren - in der Weise durchgeführt, daß man das sekundäre Alkylamin (Ausgangsamin) und den Hydrierkatalysator auf Hydriertemperatur erhitzt, Wasserstoff aufdrückt, die Aldehydverbindung einbringt und die Reaktionsmischung aus Ausgangsamin, Hydrierkatalysator und Aldehydverbindung beim eingestellten Wasserstoffdruck reagieren läßt (vergleiche Seite 11, Zeile 20 bis Zeile 8 auf Seite 12 und die Beispiele). Als Hydrierkatalysator wird unter anderen Metallen Nickel auf pulverisierter oder granulierter Kohle empfohlen, das ist ein Nickel-Trägerkatalysator mit dem speziellen Trägermaterial Kohle; auch dies steht im Gegensatz zum erfindungsgemäßen Verfahren, bei dem ein trägerloser Nickelkatalysator eingesetzt wird, nämlich Raney-Nickel. In dieser Druckschrift wird von der Verwendung von Raney-Nickel sogar abgeraten.

In FR-A-2 203 382 wird die gleiche Reaktion beschrieben, also wiederum die Umsetzung von sekundären Alkylaminen mit Aldehydverbindungen unter hydrierenden Bedingungen. Als Hydrierkatalysator wird eine Mischung aus Silber und Palladium auf einem Trägermaterial eingesetzt.

Die in Rede stehenden linearen Dialkylpolyhydroxyamine, insbesondere in Form von Dialkylglycaminen oder Dialkylglucaminen, haben in letzter Zeit immer mehr Bedeutung erhalten, da sie biologisch abbaubar sind und aus dem nachwachsenden Rohstoff Zucker gewonnen werden können. Es besteht deshalb ein verstärktes Bedürfnis nach einem Verfahren, mit dem diese Aminoalkohole in hoher Ausbeute und Reinheit erhalten werden. Mit der vorliegenden Erfindung wird ein solches Verfahren zur Verfügung gestellt.

Das erfindungsgemäße Verfahren zur Herstellung von einem tertiären Dialkylpolyhydroxyamin ist dadurch gekennzeichnet, daß man ein sekundäres N-Monoalkylpolyhydroxyamin mit einem Aldehyd im Molverhältnis von 1 zu 0,90 bis 1,5, vorzugsweise 1 zu 1,03 bis 1,1, in Wasser als Lösungensmittel umsetzt und anschließend in Gegenwart von Raney-Nickel bei einem Wasserstoffdruck von 10 bis 150 bar, vorzugsweise 30 bis 100 bar, und einer Temperatur von 70 bis 150 °C, vorzugsweise 80 bis 130 °C, zum tertiären Dialkylpolyhydroxyamin hydriert.

Dieses erfindungsgemäße Verfahren wird durch keine der vorstehend genannten Druckschriften nahegelegt, weder einzeln noch in Kombination betrachtet.

Die Umsetzung wird in zwei Reaktionsschritten durchgeführt, wobei man
a) zunächst ein sekundäres N-Monoalkylpolyhydroxyamin mit einem Aldehyd im Molverhältnis von 1 zu 0,90 bis 1,5, vorzugsweise 1 zu 1,03 bis 1,1, in Wasser als Lösungsmittel bei einer Temperatur von 15 bis 60 °C, vorzugsweise von 20 bis 40 °C, und Atmosphärendruck zum N=Monoalkylpolyhydroxyamin/Aldehyd-Addukt umsetzt und
b) das im Schritt a) erhaltene Reaktionsprodukt (das im wesentlichen aus dem gebildeten Addukt und Wasser besteht) in Gegenwart von Raney-Nickel mit Wasserstoff bei einem Druck von 10 bis 150 bar, vorzugsweise 30 bis 100 bar, und einer Temperatur von 70 bis 150 °C, vorzugsweise 80 bis 130 °C, zum tertiären Dialkylpolyhydroxyamin hydriert.

Das Wasser als Lösungsmittel wird im allgemeinen in einer solchen Menge eingesetzt, daS eine 5 bis 60gew.%ige Lösung vorliegt, vorzugsweise eine 15 bis 40gew.%ige Lösung, bezogen auf die Menge an eingesetztem N-Monoalkylpolyhydroxyamin. In der angegebenen Wassermenge ist auch jenes Wasser enthalten, das zum Beispiel bei Einsatz von Formaldehyd in Form einer vorzugsweise 30 bis 40gew.%igen wäßrigen Lösung eingebracht wird. Der metallische Hydrierkatalysator ist Raney-Nickel.

Der Hydrierkatalysator wird im allgemeinen in einer Menge von 0,1 bis 10 Gew.-% eingesetzt, vorzugsweise in einer Menge von 0,5 bis 5 Gew.-%, bezogen auf N-Monoalkylpolyhydroxyamin (die genannten Gewichtsprozentmengen beziehen sich klarerweise auf das Metall allein).

Die erfindungsgemäße Umsetzung wird im einzelnen vorzugsweise so durchgeführt, daß das N-Monoalkylpolyhydroxyamin und Wasser vorgelegt und bei Atmosphärendruck auf 15 bis 60 °C, vorzugsweise 20 bis 40 °C, gebracht werden. Zu dieser Lösung wird dann die Aldehydverbindung unter Rühren und Aufrechterhalten der genannten Temperatur kontinuierlich oder portionsweise dazugegeben, worauf man zur vollständigen Umsetzung (Adduktbildung) bei 15 bis 60 °C, vorzugsweise 20 bis 40 °C, nachreagieren läßt (0,5 bis 2 Stunden). Das so erhaltene Reaktionsprodukt, das im wesentlichen aus dem gebildeten Addukt und Wasser besteht, wird nun einer Hydrierung unterworfen. Dazu wird es gemeinsam mit Raney-Nickel bei einer Temperatur von 70 bis 150 °C, vorzugsweise 80 bis 130 °C, unter Rühren mit Wasserstoff bis zu einem Druck von 10 bis 150 bar beaufschlagt, vorzugsweise von 30 bis 100 bar. Dieser Wasserstoffdruck wird bei der genannten Temperatur und unter Rühren solange aufrechterhalten, bis kein Wasserstoff mehr aufgenommen wird, was einschließlich einer eventuellen Nachreaktion im allgemeinen 1 bis 5 Stunden dauert. Das so erhaltene Reaktionsprodukt besteht im wesentlichen aus dem gewünschten tertiären Dialkylpolyhydroxyamin und Wasser. Wenn eine Isolierung der tertiären Aminverbindung aus der Wasserlösung gewünscht wird, wird man den Katalysator und das Wasser abtrennen und so die tertiäre Aminverbindung gewinnen, gegebenenfalls durch Umkristallisieren aus einem geeigneten Lösungsmittel wie Isopropanol. Das genannte Abtrennen kann zum Beispiel durch Filtrieren und Strippen oder Destillieren durchgeführt werden.

Das erfindungsgemäße Verfahren liefert die tertiären Dialkylpolyhydroxyamine in hoher Ausbeute und Reinheit. Es kann nicht nur diskontinuierlich, sondern auch kontinuierlich durchgeführt werden.

Bezüglich der Ausgangsverbindungen sei noch folgendes gesagt: Die Aldehydverbindung ist vorzugsweise ein aliphatischer Aldehyd, beispielsweise Formaldehyd oder Acetaldehyd, wobei Formaldehyd bevorzugt ist. Formaldehyd wird vorzugsweise in Form einer 30 bis 40gew.%igen wäßrigen Lösung eingesetzt. Das N-Monoalkylpolyhydroxyamin, häufig einfach als sekundäres Polyhydroxyamin bezeichnet, ist vorzugsweise ein solches der nachstehenden Formel (1)

R¹-NH-Z (1)

worin R¹ ein C₁ bis C₁₈-Alkyl ist, vorzugsweise C₁ bis C₆-Alkyl oder C₁ bis C₆-Hydroxyalkyl wie 2-Hydroxyethyl oder 2-Hydroxypropyl, und besonders bevorzugt Methyl oder Ethyl ist und Z ein Polyhydroxykohlenwasserstoffrest mit einer linearen Kohlenwasserstoffkette mit mindestens 3 OH oder ein alkoxyliertes Derivat davon ist, vorzugsweise ein ethoxyliertes und/oder propoxyliertes Derivat. Z ist vorzugsweise der Rest einer Mono-, Oligo- oder Polysaccharidverbindung, die über eine Methylengruppe mit dem Stickstoffatom verbunden ist. Z ist besonders bevorzugt eine Gruppe der Formel -CH₂-CH(OH)ₙ-CH₂OH, worin n eine ganze Zahl von 3 bis 5 ist, vorzugsweise 3 oder 4 und besonders bevorzugt 4 ist. Die Ausgangsprodukte der Formel (1) sind bekannt und im Handel erhältlich.

Die nach dem erfindungsgemäßen Verfahren hergestellten tertiären Polyhydroxyamine entsprechen also der nachstehenden Formel (2) 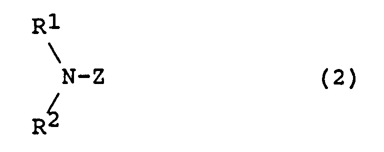 worin R¹ und Z die angegebenen Bedeutungen haben und R² der durch die Aldehydverbindung eingeführte Substituent ist. Aus den obigen Ausführungen folgt, daß die Di-C₁ bis C₆-alkylglycamine die bevorzugten Verbindungen sind, wobei die Glucaminverbindungen wie Dimethylglucamin, Diethylglucamin und Ethylmethylglucamin besonders bevorzugt sind.

Die Erfindung wird nun an Beispielen noch näher erläutert.

### Beispiel 1

In 1500 g von einer 43gew.%igen wäßrigen N-Methylglucaminlösung (Aminzahl 22,3) werden 288,7 g von einer 36,5gew.%igen wäßrigen Formaldehydlösung bei etwa 15 °C zudosiert. Das Gemisch wird 1 Stunde bei etwa 15 °C gerührt und anschließend mit 28 g Raney-Nickel versetzt und bei 100 °C und 30 bar Wasserstoffdruck hydriert.
Ausbeute an N,N-Dimethylglucamin: 98,2 %; Schmelzpunkt: 90 °C

### Beispiel 2

In 1500 g von einer 43gew.%igen wäßrigen N-Methylglucaminlösung (Aminzahl 22,3) werden 288,7 g von einer 36,5gew.%igen wäßrigen Formaldehydlösung bei etwa 35 °C zudosiert, worauf das Gemisch unmittelbar nach beendeter Zugabe mit 28 g Raney-Nickel versetzt und bei 100 °C und 30 bar Wasserstoffdruck hydriert wird.
Ausbeute an N,N-Dimethylglucamin: 98,1 %; Schmelzpunkt: 89 °C

### Beispiel 3

In 1500 g von einer 43gew.%igen wäßrigen N-Methylglucaminlösung (Aminzahl 22,3) werden 288,7 g von einer 36,5gew.%igen wäßrigen Formaldehydlösung bei etwa 35 °C zudosiert. Das Gemisch wird 1 Stunde bei etwa 35 °C gerührt und anschließend mit 28 g Raney-Nickel versetzt und bei 130 °C und 100 bar Wasserstoffdruck hydriert.
Ausbeute an N,N-Dimethylglucamin: 97,8 %; Schmelzpunkt: 90 °C

### Beispiel 4

In eine Mischung aus 689 g von einer 43gew.%igen wäßrigen N-Methylglucaminlösung (Aminzahl 22,3) und 811 g zusätzlichem Wasser werden 132 g von einer 36,5gew.%igen wäßrigen Formaldehydlösung bei etwa 35 °C zudosiert. Das Gemisch wird 1 Stunde bei etwa 35 °C gerührt und anschließend mit 28 g Raney-Nickel versetzt und bei 100 °C und 30 bar Wasserstoffdruck hydriert.
Ausbeute an N,N-Dimethylglucamin: 97,8 %; Schmelzpunkt: 90 °C

### Beispiel 5

In 1500 g von einer 43gew.%igen wäßrigen N-Methylglucaminlösung (Aminzahl 22,3) werden 288,7 g von einer 36,5gew.%igen wäßrigen Formaldehydlösung bei 60 °C zudosiert. Das Gemisch wird 1 Stunde bei etwa 60 °C gerührt und anschließend mit 28 g Raney-Nickel versetzt und bei 100 °C und 30 bar Wasserstoffdruck hydriert.
Ausbeute an N,N-Dimethylglucamin: 98,4 %; Schmelzpunkt: 91 °C

### Beispiel 6

40 g N-Butylglucamin (Aminzahl 41,9) werden in 60 g Wasser suspendiert. Zur Suspension werden bei etwa 25 °C 14,8 g von einer 36,5gew.%igen wäßrigen Formaldehydlösung zugesetzt. Es entsteht eine klare Lösung, die noch 1 Stunde bei etwa 25 °C gerührt wird. Anschließend wird die Lösung mit 1,7 g Raney-Nickel versetzt und bei 100 °C und 30 bar Wasserstoffdruck hydriert.
Ausbeute an N-Methyl-N-butylglucamin: 99,3 %;
Schmelzpunkt: 52 °C

### Beispiel 7

40 g N-Hexylglucamin (Aminzahl 37,9) werden in 60 g Wasser suspendiert. Zur Suspension werden bei 25 °C 13,1 g von einer 36,5gew.%igen wäßrigen Formaldehydlösung zugesetzt. Es entsteht eine klare Lösung, die noch 1 Stunde bei 25 °C gerührt wird. Anschließend wird die Lösung mit 1,9 g Raney-Nickel versetzt und bei 100 °C und 30 bar Wasserstoffdruck hydriert.
Ausbeute an N-Methyl-N-hexylglucamin: 99,2 %;
Schmelzpunkt: 74 °C

## Patentansprüche

1. Verfahren zur Herstellung von tertiären Dialkylpolyhydroxyaminen, dadurch gekennzeichnet, daß man
a) zunächst ein sekundäres N-Monoalkylpolyhydroxyamin mit einem Aldehyd im Molverhältnis von 1 zu 0,9 bis 1,5 in Wasser als Lösungsmittel bei einer Temperatur von 15 bis 60 °C und Atmosphärendruck zum N-Monoalkylpolyhydroxyamin/Aldehyd-Addukt umsetzt und
b) das im Schritt a) erhaltene Reaktionsprodukt in Gegenwart von Raney-Nickel als Hydrierkatalysator mit Wasserstoff bei einem Druck von 10 bis 150 bar und einer Temperatur von 70 bis 150 °C zum tertiären Dialkylpolyhydroxyamin hydriert.

2. Verfahren nach Anspruch 1, dadurch gekennzeichnet, daß man im Schritt a) das sekundäre N-Monoalkylpolyhydroxyamin und den Aldehyd in einem Molverhältnis von 1 zu 1,03 bis 1,1 einsetzt und bei einer Temperatur von 20 bis 40 °C zum Addukt umsetzt und im Schritt b) das Addukt bei einem Wasserstoffdruck von 30 bis 100 bar und einer Temperatur von 80 bis 130 °C hydriert.

3. Verfahren nach Anspruch 1 oder 2, dadurch gekennzeichnet, daß man ein sekundäres N-Mono-C₁ bis C₆-alkylglycamin und Formaldehyd oder Acetaldehyd einsetzt.

## Claims

1. A process for the preparation of tertiary dialkylpolyhydroxy-amines, comprising
a) reacting a secondary N-monoalkylpolyhydroxy-amine in the first step with an aldehyde in a molar ratio of 1 : from 0.9 to 1.5 in water as solvent at a temperature of from 15 to 60°C and at atmospheric pressure to give the N-monoalkylpolyhydroxy-amine/aldehyde adduct, and
b) hydrogenating the reaction product obtained in step a) in the presence of Raney nickel as hydrogenation catalyst with hydrogen at a pressure of from 10 to 150 bar and at a temperature of from 70 to 150°C to give the tertiary dialkylpolyhydroxy-amine.

2. The process as claimed in claim 1, wherein in step a) the secondary N-monoalkylpolyhydroxy-amine and the aldehyde are employed in a molar ratio of 1 : from 1.03 to 1.1 and are reacted at a temperature of from 20 to 40°C to give the adduct, and in step b) the adduct is hydrogenated at a hydrogen pressure of from 30 to 100 bar and at a temperature of from 80 to 130°C.

3. The process as claimed in claim 1 or 2, wherein a secondary N-mono-C₁ to C₆-alkylglycamine and formaldehyde or acetaldehyde are employed.

## Revendications

1. Procédé pour la préparation de dialkylpolyhydroxylamines tertiaires caractérisé en ce que
a) on fait réagir d'abord une N-monoalkylpolyhydroxylamine secondaire avec un aldéhyde dans un rapport molaire de 1 à 0,9 à 1,5 dans l'eau comme solvant, à une température de 15 à 60 °C, et à la pression atmosphérique, pour obtenir un produit d'addition de N-monoalkylpolyhydroxylamine/aldéhyde, et
b) on hydrogène le produit de réaction obtenu à l'étape a) en présence de nickel de Raney en tant que catalyseur d'hydrogénation, avec de l'hydrogène à une pression de 10 à 150 bars, et à une température de 70 à 150 °C, pour obtenir la dialkylpolyhydroxylamine tertiaire.

2. Procédé selon la revendication 1, caractérisé en ce que l'on fait réagir dans l'étape a) la N-monoalkylpolyhydroxylamine secondaire et l'aldéhyde dans un rapport molaire de 1 à 1,03 à 1,1, et à une température de 20 à 40 °C pour obtenir le produit d'addition, et on hydrogène à l'étape b) le produit d'addition à une pression d'hydrogène de 30 à 100 bars, et une température de 80 à 130 °C.

3. Procédé selon la revendication 1 ou 2, caractérisé en ce que l'on utilise une N-mono(alkyl en C₁-C₆)-glycamine secondaire et le formaldéhyde ou l'acétaldéhyde.
